# EUROPEAN PATENT SPECIFICATION

(11) **EP 2 133 115 B1**
(45) Date of publication and mention of the grant of the patent: **13.04.2016**
(21) Application number: 09162602.8
(22) Date of filing: 12.06.2009
(51) Int. Cl.: A61M 25/00, A61M 25/06, A61M 29/00

(54) **Guide sheath dilator**
Führungshüllendilatator
Dilatateur de gaine de guide

(30) Priority: 12.06.2008 US 138039
(43) Date of publication of application: 16.12.2009
(60) Divisional application: 12171909.0
(73) Proprietor: Terumo Medical Corporation, Somerset, New Jersey 08873 (US)
(72) Inventor: Trask, Linda, Newark, DE 19711 (US); Ferguson, Frank, Belle Mead, NJ 08502 (US); Anderson, Susan, New Hope, PA 18938 (US)
(74) Representative: Jenkins, Peter David

(56) References cited:
- US-A- 5 098 392
- US-A- 5 300 048
- US-A- 5 639 276
- US-A- 6 036 682

## Description

### BACKGROUND OF THE INVENTION

### Field of the Invention

The present invention relates to a dilator. Specifically, the present invention relates to a selective guide sheath dilator with multiple stiffness sections.

### Background of the Invention

In today's medical field, many medical procedures require entry into a patient's blood vessel for purposes of accessing a desired site, e.g., angioplasty and stenting. In order to gain access to the desired site, a sheath is usually advanced through the blood vessel. Once in place within the patient's vessel, various types of medical instrumentation can be fed through the sheath and positioned at the desired site so that the procedure may be performed.

To initially gain access to a particular site within a patient, a needle is used to puncture the patient's skin and gain entry to a desired blood vessel. A guide wire is then inserted into a lumen in the needle and is fed into the blood vessel. The needle is then removed, with the guide wire being left in place.

A dilator/sheath assembly is then placed over the guide wire and advanced to a position inside the blood vessel. The distal end of the dilator may be tapered to a relatively small diameter and extend beyond the distal end of the sheath. The tapered distal end of the dilator allows the dilator/sheath assembly to be introduced into a patient's vessel in a manner that gradually increases the size of the opening in the vessel so that the vessel can ultimately accommodate the larger sized sheath without causing trauma, injury or other difficulties to the patient.

Once the guide wire and the dilator/sheath assembly are advanced within the blood vessel to the desired site, the dilator is removed. The guide wire and sheath are now used to introduce and guide medical instrumentation to the desired site within the blood vessel.

The disadvantage of the above system and method is that the dilator typically used as a mechanism for guiding the sheath into a blood vessel is made from a uniform material designed primarily for the initial access. Often the dilator must be replaced with a more flexible, shaped catheter to track the sheath through very torturous anatomy to the desired site in the vessel. A need thus exists for a dilator that has flexibility but retains the rigidity needed for advancement of the sheath assembly.

US5639276 describes a device and method for rapid, atraumatic placement of medical devices in the right ventricle of a patient. The device includes an introducer sheath of varied stiffness along its length, being more flexible towards its distal end and more stiff towards its proximal end. The device includes a dilator located within the introducer sheath that is removable from the introducer sheath after the sheath has been sufficiently inserted into the patient.

### SUMMARY OF THE INVENTION

The present invention provides an assembly of a dilator and a sheath, the assembly being as defined in Claim 1.

The dilator of embodiments of the present invention offers multiple stiffness sections. Specifically, this dilator includes a shaft that has a distal end, a proximal end and at least two stiffness sections. These stiffness sections become less rigid and may taper as they approach the distal end.

The stiffness sections may be fused together thereby creating varying stiffness and/or flexibility in the dilator. The dilator further includes a lumen that traverses an interior of the shaft from the proximal end to the distal end as well as a hub that is located on the proximal end of the shaft.

The dilator may also include an atraumatic tip (i.e., a tip that does not cause trauma or damage) that may be connected to the distal end of the shaft. If this tip is used, the tip preferably would be the softest and least rigid section of the shaft.

In one embodiment, the dilator has three sections. The preferred lengths for each of these sections are as follows: the first stiffness section maybe approximately 25-90 cm, the second stiffness section may be approximately 1-70 cm and the third stiffness section may be approximately 1-70 cm. In most cases, the third stiffness section would start from approximately 15 cm from a distal end of the sheath and extend beyond the distal tip of the sheath.

The dilator may also contain one or more radiopaque markers in the distal area to improve visibility under fluoroscopy and possibly be used for measurement approximations. The dilator may also have a shaped distal end to allow easier vessel selection. Further, the distal end may have apertures located near the tip of the dilator so as to deliver contrast media to a work-destination area.

Each stiffness section of the dilator may be manufactured from different material blends and/or grades. In the preferred embodiments, the material of the stiffness sections are preferably manufactured of polypropylene (PP) and elastomer and may contain sufficient radiopaque filler to allow visibility under fluoroscopy. It is noted that other medical grade materials that retain there shape while being displaced within the vascular system, such as TPE, homopolymers and copolymers, may be used as a substitute for one of the above materials.

In one embodiment, the three sections maybe manufactured with (1) the first stiffness section being be made from a blend of between 20% and 80% high performance elastomer (TPV) and between 20% and 80% PP impact copolymer (preferably 50% of each), (2) the second stiffness section being be made from a blend of between 20% to 60% high performance elastomer (TPV) and 40% to 80% PP impact copolymer (preferably a 60/40 high performance elastomer (TPV)/PP impact copolymer blend) and (3) the third stiffness section being be made from between 0% to 25% PP impact copolymer and 75% to 100% high performance elastomer (TPV) (preferably 100% high performance elastomer (TPV) ).

A guide wire may extend through the lumen of the dilator and be used as a guide for advancing the assembly. In a preferred embodiment, the guide wire is a 0.0965cm (.038") diameter stainless steel or nitinol type wire of appropriate length.

The sheath also has a distal end, a proximal end and a lumen. The dilator extends through the lumen of the sheath optionally with the distal end of the dilator extending beyond the distal end of the sheath. The sheath, when used at a femoral access point, may be approximately 90cm in length and connected to a housing provided with a hemostatic valve. A rotatable cap may also be operatively associated with the hemostatic valve for operating the valve upon rotation of the cap.

A method of using the sheath assembly comprises the steps of: inserting a needle into a patient to puncture a patient's skin and gain entry to a desired blood vessel; inserting a guide wire into a lumen in the needle; feeding the guide wire into the blood vessel; removing the needle and leaving the guide wire in place; placing a dilator that is generally coaxially disposed within a sheath over the guide wire, the dilator having a distal end, a proximal end and a lumen, the dilator having at least two stiffness sections, the stiffness sections becoming less rigid as they approach the distal end, the dilator tapering from the proximal end to the distal end; advancing the guide wire, followed by the dilator followed by the sheath through vascular to a specific point; removing the dilator when the specific point is reached; performing a procedure at the specific point using tools that are advanced through the sheath.

The dilator may be used to assist in procedures such as angioplasty and stenting by, e.g., accessing the vascular system beyond the aortic arch from a femoral access point or accessing a lower extremity on a patient (an area near and/or below the knee) via a femoral artery. The latter uses the dilator to extend downward from the access point to an area near the knee or to extend above the access point around the iliac arch and down towards near and/or below the knee of the other leg.

Trauma caused by the access is reduced due to the varying stiffnesses of the dilator because the stiffnesses allow the dilator to move easier through the vasculature. When the dilator is removed from the sheath, the sheath retains a uniform rigidity which allows for placement of medical equipment at the distal end of the sheath.

### BRIEF DESCRIPTION OF THE DRAWINGS

These and other features, aspects, and advantages of the apparatus and methods of the present invention will become better understood with regard to the following description and accompanying drawings where:
Figure 1 is a prospective view of an unassembled sheath assembly according to a first embodiment of the present invention;
Figure 2 is a prospective view of a dilator according to a second embodiment of the present invention;
Figure 3 is a prospective view of a dilator according to a third embodiment of the present invention;
Figure 4 is a prospective view of an assembled sheath assembly according to a first embodiment of the present invention; and
Figures 5a-b are side views of a distal end of the dilator having apertures.

### DETAILED DESCRIPTION OF THE INVENTION

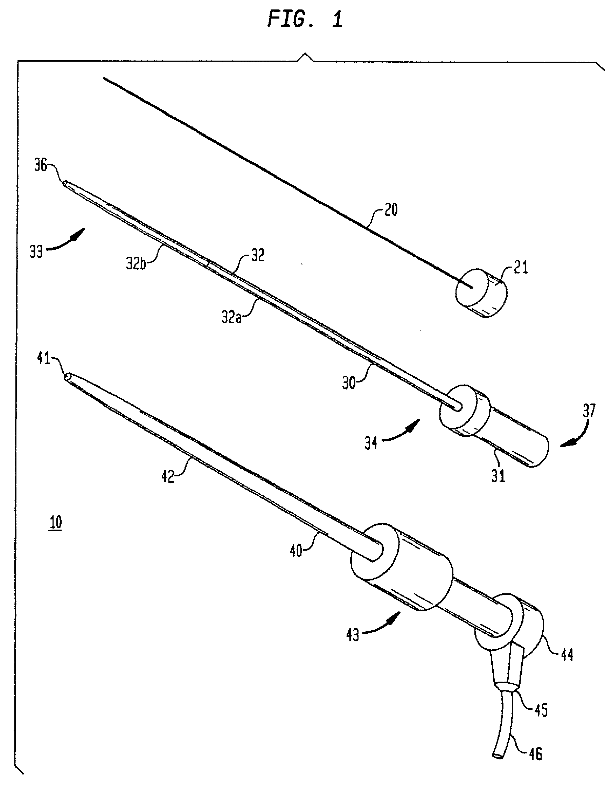

The present invention enhances the characteristics of dilators by providing a dilator that has multiple stiffness sections meaning that each section of the dilator has a different durometer hardness. FIG. 1 illustrates an unassembled sheath assembly 10 that includes the dilator 30 of the present invention as well as a guide wire 20 and a sheath device 40.

For the guide wire 20 to first access a blood vessel, an insertion tool known in the art, but not shown, is used. The insertion tool has a puncture needle. The puncture needle punctures an appropriate point in the vascular vessel. The guide wire 20 is then inserted through the needle and into the vascular vessel. Once inside the vessel, a distal end of the guide wire 20 is manipulated by a push-pull and turn technique by hand or with a torque device at an operations assembly 21 located on the proximal end of the guide wire 20.

To achieve a smooth manipulation when inserting the guide wire 20 into the vascular vessel, it is required for the guide wire 20 to have certain multi-mechanical properties. The multi-mechanical properties include flexibility, the ability to remain straight in an unrestricted free state and good restitution when returning from a deformed state.

The guide wire 20 of this type is also preferred at its distal end portion to have high flexibility properties, while at the same time, preferred at its proximal portion to have functionally gradient rigidity. The distal end should have high maneuverability to properly respond to the manipulation which is to be done at the operations assembly 21. In a preferred embodiment, the guide wire 20 is a 0.0965cm (.038") diameter stainless steel or nitinol type wire of appropriate length but any size guide wire may be used.

The dilator device 30 is comprised of an elongated tubular shaft/dilator 32 and a hub 31. The elongated tubular shaft 32 is used to access a vessel while the hub 31 is used to interact with the sheath 40.

The dilator 32 and the hub 31 are fixedly secured to one another in a manner that prevents axial and rotational movement of the dilator 32 relative to the hub 31. Both the dilator 32 and the hub 31 are provided with a centrally extending lumen, the lumen traversing an interior of the dilator 32 from a distal end 36 and a proximal end 37, with the lumen's distal end 36 in the dilator 32 communicating with the lumen's proximal end 37 in the hub 31. The lumen's proximal end 37 in the hub 31 is open at the proximal end of the hub 31 while the lumen's distal end 36 in the dilator 32 opens to the distal end of the dilator 32. Thus, the lumen extends completely through the dilator device 30 and opens to the outside at opposite ends of the dilator device 30.

In addition, as can be seen from FIG. 1, the first embodiment of the dilator device 30 of the present invention offers multiple stiffness sections 32a-b. That is, the dilator device 30 includes a shaft 32 that has a distal end 33, a proximal end 34 and two stiffness sections 32a-b. These stiffness sections 32a-b become less rigid as they approach the distal end 33. In other words, the shaft is the softest at the distal end (approximately 30 shore D to 70 shore D) and transitions to the stiffest section at the proximal end (approximately 50 shore D to 80 shore D). The varying stiffness sections 32 a-b allow the dilator device 30 to slightly bend around twisted and bent vasculature, so as the dilator 32 is advanced and the stiffness is changed, the bent vasculature is straightened. The varying stiffness sections also provide for greater pushability when advancing through the vasculature.

The dilator 32 may also taper along the length the dilator 33. That is, the dilator may taper along the full length of the dilator or the dilator may taper between two specific points along the dilator. For example, the dilator may taper from the proximal end to an approximately the midpoint of the dilator or the dilator may taper from a finite distance from the proximal tip to a finite distance from the distal tip.

The dilator may also be provided in a pre-shaped form to allow easier vessel selection. These shapes may include, but are not limited to, straight, angle, angle taper, multipurpose, cobra, simmons, headhunter, mani, JB, hockey stick, LIMA, RDC, vertebral, J-curve and vitek

The stiffness sections may vary in length based upon application as well as the length of the sheath 40 to be utilized. In a preferred embodiment, a 90 cm sheath is used which usually results in a dilator 32 that is at least 95 cm in length. The dilator 32 has two stiffness sections 32 a-b with the first stiffness section 32a being approximately 25-90 cm in length and the second stiffness section 32b be approximately 1-70 cm in length. Usually, the second stiffness section 32b starts from approximately 15 cm from a distal end of the sheath 40 and will extend beyond the distal tip of the sheath 40.

The material blends of these stiffness sections vary significantly depending on application and length of the dilator. The stiffness sections 32a-b can be made from a blend of between 0-95% PP impact copolymer and between 5-100% high performance elastomer (TPV). In a preferred embodiment, the first section is about 50/50 high performance elastomer (TPV)/PP impact copolymer blend and the second section is about 20/80 PP impact copolymer/ high performance elastomer (TPV) blend. In this embodiment, high performance elastomer (TPV) and PP impact copolymer were used but other polypropylene and elastomer choices can be substituted for the high performance elastomer (TPV)/PP impact copolymer blend. Such choices may include polypropylene and impact polypropylene copolymers, thermoplastic elastomers (e.g., TPO, TPU, TPV, polyester elastomers (Hytrel), polyamide elastomers (PEBAX), and the like), nylon, polyethylene, fluoropolymer (FEP, PFA, ETFE and the like), homopolymers, copolymers, ect.

The dilator may also contain one or more radiopaque markers in the distal area to improve visibility under fluoroscopy and possibly be used for measurement approximations. The radiopaque markers may be formed from bismuth subcarbonate or barium sulfate, as well as additional colorants.

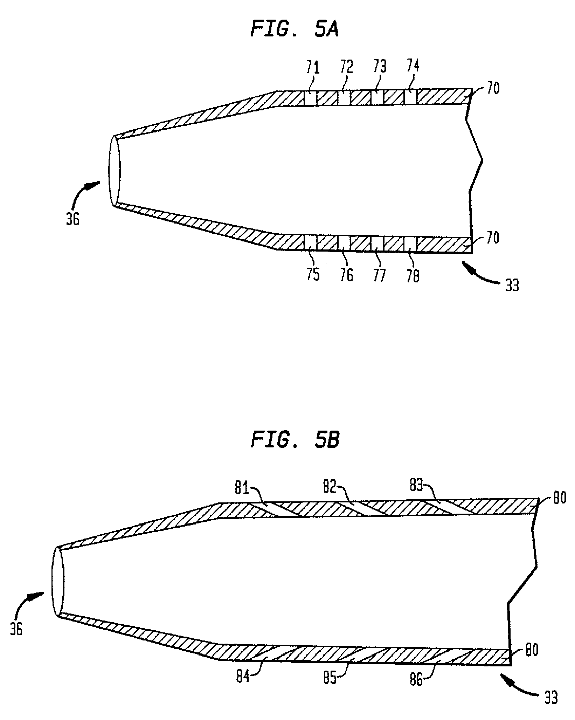

As shown in Figs. 5a-b, the distal end 33 of the shaft 32 may also include straight apertures 71-78 and/or angled apertures 81-86 that traverse the dilator tubing wall 70, 80. These straight and angled apertures 71-78 and 81-86 may assist in delivering contrast media to the site where a medical procedure is to be performed by visually enhancing the site in which a practitioner will work as well as aspiration of the work site. The number, angle and size of the apertures will vary depending on the procedure being performed and other varied aspects of the dilator and work site.

The stiffness sections 32a-b are usually fused/joined together with RF energy or a hot air gun, thereby creating varying stiffness and/or flexibility in the dilator 30, although other methods of joining the materials may be used.

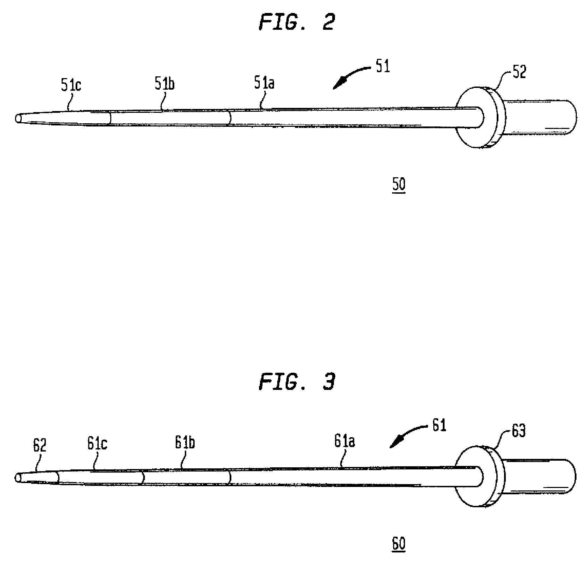

In a second embodiment, as shown in Fig. 2, the shaft 51 may have three stiffness sections 51 a-c. The first stiffness section 51a may be approximately 25-90 cm in length with a durometer of approximately 50 shore D to 80 shore D, the second stiffness section 51b may be approximately 1-70 cm in length with a durometer of approximately 30 shore D to 70 shore D and the third stiffness section 51c may be approximately 1-70 cm in length with a durometer of approximately 40 shore A to 40 shore D.

The material blends of these stiffness sections also vary significantly depending on application and length of the dilator. The stiffness sections 51a-c can also be made from a blend of between 0-95% PP impact copolymer and between 5-100% high performance elastomer (TPV). In a preferred embodiment, the first stiffness section 51 a may be made from an approximate blend of 50% high performance elastomer (TPV) and 50% PP impact copolymer containing approximately 30% Bi₂SO₄, the second stiffness section 51 b may be made from an approximate blend of 60% PP impact copolymer and 40% high performance elastomer (TPV) containing approximately 30% Bi₂SO₄ and the third stiffness section 51c may be made from approximately 100% high performance elastomer (TPV) containing approximately 30% Bi₂SO₄.

In a third embodiment, as shown in Fig. 3, a tip 62 can be connected to the distal end of the shaft 61. The tip would be the softest and least rigid section of the shaft 61 resulting in an atraumatic tip 62. In this embodiment, the tip is attached to a dilator 60 having three stiffness sections 61a-c. But the tip may be used in conjunction with a shaft having any number of stiffness sections including a shaft that has a substantially even gradient of stiffness.

The dilators 30, 50 and 60 are all used in conjunction with the sheath device 40. These dilators 30, 50 and 60 are coaxially disposed within a lumen 41 of the sheath device 40. The sheath 40 is approximately 90 cm long when used with a femoral access point but longer (approximately 120 cm) and shorter (approximately 30 cm) sheaths and dilators may be used when accessing a vessel.

The sheath device 40 includes an elongated tubular sheath 42, a hub 43 and a valve 44. The sheath 42 can be secured to the hub 43 by way of a fitting provided at the proximal end of the sheath 42. The proximal end portion of the hub 43 is provided with an externally threaded portion (not shown) that may be threadably engaged by internal threads (not shown) on the valve 44. The valve 44 can thus be rotated relative to the hub 43, thus rendering the valve removable from the sheath/hub assembly.

The valve 44 is also preferably provided with an integrally formed branch port 45 connected to a tubing 46. The tubing can 46 be connected to a three-way stop cock (not shown) to allow fluids to be injected into or withdrawn from the patient during the procedure. For example, the tubing 46 and the stop cock can be used to sample blood or inject a contrast agent in conjunction with the apertures shown in Figs 5a-b.

The elongated sheath 42 and the hub 43 together are both provided with a lumen 41. The tubing 46 also communicates with the lumen 41. The lumen 41 opens to the proximal end of the hub 43 and opens to the distal end of the sheath 42.

Another embodiment for the valve 44 may be a housing (not shown). The housing may be provided with a hemostatic valve (not shown). The hemostatic valve is in the form of a cylindrical plastic element having a generally centrally located through hole. The hemostatic valve is positioned within a chamber in the housing. The valve has a slit style opening that allows a catheter or other device to be inserted through it and hugs the inserted device to provide hemostatis. The rotatable cap 44 is operatively associated with the hemostatic valve. That is, by rotating the cap 44 so that the cap 44 moves axially with respect to the hub 43 in a direction towards the elongated sheath 42, an internal portion of the cap 44 engages the cylindrical element forming the hemostatic valve and thus compresses the cylindrical element causing the through hole in the cylindrical member to be closed. Thus, by suitably rotating the cap 44 on the hub 43, the hemostatic valve can be moved between an opened position and a closed position.

The hemostatic valve can also be used to prevent blood loss during a procedure involving use of the sheath assembly 40 by providing a tight seal around the outer surface of the dilator device 30. The hemostatic valve also allows dilators of different sizes and profiles to pass through the hemostatic valve. And the elongated sheath 40 is adapted to be inserted into the lumen in the valve and then advanced so that the elongated sheath 40 extends through the lumen in the valve.

In addition, the tapered distal end portion of the dilator device 30 extends beyond the distal end of the elongated sheath 40 such that a smooth transition exists between the tapered distal end portion of the dilator 30 and the distal end portion of the sheath 40. It is worthy to note that the majority of the dilator 30 is stiff to allow support of the sheath 40 as the sheath 40 is advanced through vascular system. And when the dilator 30 is removed from the sheath 40, the sheath 40 retains a substantially uniform and consistent rigidity throughout its length.

Further, the distal end portion of the sheath 40 can be configured as a formed tip (e.g., having a slightly tapered end) to further facilitate the smooth transition between the tapered distal end portion of the dilator 30 and the distal end portion of the sheath 40. To maintain contact between the sheath 40 and the dilator 30, a retaining device, such as disclosed in U.S. Pat. No. 6,719,772, may be used.

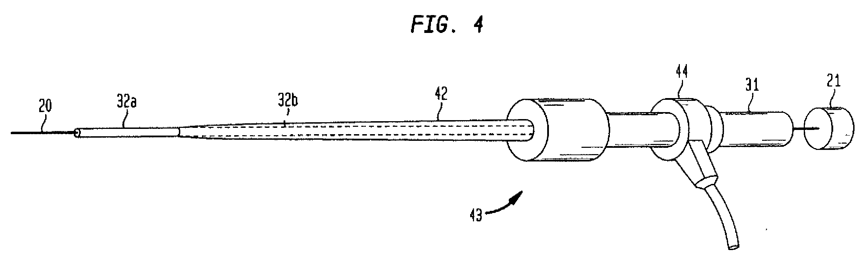

To assemble the guiding sheath assembly 10, the elongated dilator 30 is inserted into the open end of the valve. The elongated dilator 30 is then advanced so that the elongated dilator 30 extends through the lumen in the sheath. In an assembled state, as shown in Figure 4, the tapered distal end portion of the dilator 30 extends beyond the distal end of the elongated sheath 40 such that a smooth transition exists between the tapered distal end portion of the dilator 30 and the tapered distal end portion of the sheath 40.

To use the sheath assembly 10, a needle is first used to puncture a patient's skin and gain access to the patient's vessel. This is typically a blood vessel but may be others types of body vessels. Once access to the blood vessel has been gained with the needle, a guide wire 20 is inserted into the needle and is positioned in the blood vessel. The needle can then be removed. The sheath assembly 10 is then positioned over the guide wire 20, with the guide wire 20 passing through the lumen in the dilator 30 and the hub 31. In this way, the sheath assembly 10 can be introduced into the vessel. The guide wire 20 is then fed to the desired site within the blood vessel. The sheath assembly 10 is then advanced over the guide wire 20 to the desired location.

During introduction of the sheath assembly 10 into the vessel, the exposed tapered distal end portion of the dilator 30 allows the size of the opening into the vessel to be gradually increased so that the vessel is ultimately able to accommodate the larger diameter size of the sheath 40. In addition, the smooth transition between the tapered distal end portion of the elongated dilator 30 and the formed distal end portion of the sheath 40 facilitates a smooth introduction of the distal end portion of the sheath assembly. Further, the varying stiffness sections allow the dilator to slightly bend around twisted and bent vasculature so as the dilator is advanced and the stiffness is changed, the bent vascular is straightened allowing the sheath to be unimpeded during its advancement through the vessel. Another benefit of the varying stiffness is that the stiffness is transitioned smoothly for easier sheath advancement and tracking over the wire.

Once the distal end of the elongated sheath 40 is located at the desired position in the vessel (e.g., by identifying the location of a radiopaque marker embedded within the distal end of the elongated sheath 40 or dilator device 30), the dilator device 30 comprised of the elongated dilator 30 and the hub 31 is axially withdrawn from the sheath assembly. The elongated sheath 40 and the guide wire 20 are thus left in place in the blood vessel. The elongated sheath 40 can then be used, for example, as a guiding sheath or introducer for guiding or introducing various medical instrumentation into the vessel at the desired site.

The main purpose of the dilator device 30 of the present invention includes but is not limited to accessing the vasculature beyond the aortic arch from a femoral access point and the stiffness sections allowing the dilator 30 to move easier through the vascular as well as straightening any vasculature that is bent or twisted. An additional application may be accessing a lower extremity on a patient (an area near and/or below the knee) via a femoral artery. The dilator may be used to extend downward from the access point or may extend above the access point around the iliac arch and down towards near and/or below the knee (e.g. the popletiel artery) of the other leg.

It is intended that the invention be not limited to the exact forms described and illustrated, but should be constructed to cover all modifications that may fall within the scope of the appended claims.

## Claims

1. An assembly comprising:
a dilator (30, 50, 60) comprising a shaft (32, 51, 61) having a distal end (33) and a proximal end (34), the shaft (32, 51, 61) having at least two stiffness sections (32a, 32b, 51a-c, 61a-c), a lumen traversing the shaft (32, 51, 61) from the proximal end (34) to the distal end (33), and a hub (31) located on the proximal end (34) of the shaft (32, 51, 61);
and
a sheath (40) having a distal end, a proximal end, and a lumen;
wherein the dilator (30, 50, 60) extends through the lumen of the sheath (40);
**characterised in that** the dilator (30, 50, 60) is removable from the sheath (40) such that, when the dilator (30, 50, 60) is removed from the sheath, the sheath (40) retains a uniform and consistent rigidity throughout its length; and **in that**
the stiffness sections becomes less rigid as they approach the distal end (33).

2. The assembly as claimed in Claim 1 wherein the shaft (32, 51, 61) of the dilator (30, 50, 60) tapers at a point along the shaft (32, 51, 61) to the distal end (33).

3. The assembly as claimed in Claim 1 or Claim 2 wherein the distal end of the shaft (32, 57, 61) of the dilator (30, 50, 60) contains at least one radiopaque marker.

4. The assembly as claimed in any foregoing Claim wherein the distal end of the shaft (32, 51, 61) of the dilator (30, 50, 60) is shaped to allow for easier vessel selection.

5. The assembly as claimed in any one of Claims 1 to 4 wherein the shaft (32, 51, 61) of the dilator (30, 50, 60) has a first stiffness section (51a, 61a), a second stiffness section (51b, 61b) and a third stiffness section (51c, 61c).

6. The assembly as claimed in Claim 5 wherein the third stiffness section (51c, 61c) starts from 15 cm from a distal end of the sheath (40) and extends beyond the distal end of the sheath (40).

7. The assembly as claimed in Claim 5 or Claim 6 wherein (1) the first stiffness section (51a, 61 a) is made from a blend of 0-95% of a polypropylene impact copolymer and 5-100% of high performance elastomer, (2) the second stiffness section (51b, 61b) is made from a blend of 0-95% of a polypropylene impact copolymer and 5-100% of high performance elastomer and (3) the third stiffness section (51c, 61c) made from 0-95% of a polypropylene impact copolymer and 5-100% of high performance elastomer.

8. The assembly as claimed in any one of Claims 5 to 7 wherein (1) the first stiffness section (51a, 61a) is 25-90 cm, (2) the second stiffness section (51b, 61b) is 1-70 cm and (3) the third stiffness section (51c, 61c) is 1-70 cm.

9. The assembly as claimed in any foregoing Claim wherein the stiffness sections (32a, 32b, 51 a-c, 61 a-c) are joined together thereby creating varying stiffness and/or flexibility in the dilator (30, 50, 60).

10. The assembly as claimed in Claim 9 wherein the stiffness sections (32a, 32b, 51a-c, 61a-c) are fused with RF energy.

11. The assembly as claimed in any foregoing Claim wherein the shaft (32, 51, 61) of the dilator (30, 50, 60) has a softest section at the distal end (33) and transitions to a stiffest section at the proximal end (34).

12. The assembly as claimed in any foregoing Claim wherein the stiffness sections (32a, 32b, 51a-c, 61a-c) have different material blends and/or grades.

13. The assembly as claimed in any foregoing Claim further comprising:
a tip (62) connected to the distal end (33) of the shaft (32, 51, 61) of the dilator (30, 50, 60).

14. The assembly as claimed in any foregoing Claim further comprising:
at least one aperture (71-78, 81-86) located on the distal end (33) of the shaft (32, 51, 61) of the dilator (30, 50, 60), the at least one aperture assisting in delivering contrast media to the distal end.

15. The assembly as claimed in any foregoing claim comprising a guide wire (20).

16. The assembly as claimed in any foregoing claim wherein the distal end (33) of the dilator (30, 50, 60) extends beyond the distal end of the sheath (40).

17. The assembly as claimed in any foregoing claim wherein the lumen of the dilator (30, 50, 60) traverses the dilator (30, 50, 60) from the proximal end to the distal end (33).

18. The assembly as claimed in any one of Claims 15 to 17 wherein the guide wire (20) extends through the lumen of the dilator (30, 50, 60).

19. The assembly as claimed in any one of Claims 15 to 18 wherein the guide wire (20) is a 0.0965cm (.038") diameter stainless steel or nitinol type wire.

20. The assembly as claimed in any one of the foregoing claims wherein the sheath (40) is 5cm - 120cm in length.

## Patentansprüche

1. Anordnung, die Folgendes umfasst:
einen Dilatator (30, 50, 60), der einen Schaft (32, 51, 61) mit einem distalen Ende (33) und einem proximalen Ende (34), wobei der Schaft (32, 51, 61) mindestens zwei Steifheitsabschnitte (32a, 32b, 51a-c, 61a-c) aufweist, ein Lumen, das durch den Schaft (32, 51, 61) von dem proximalen Ende (34) zu dem distalen Ende (33) verläuft, und einen Ansatz (31), der sich an dem proximalen Ende (34) des Schafts (32, 51, 61) befindet, umfasst;
und
eine Hülse (40) mit einem distalen Ende, einem proximalen Ende und einem Lumen;
wobei der Dilatator (30, 50, 60) sich durch das Lumen der Hülse (40) erstreckt;
**dadurch gekennzeichnet, dass** der Dilatator (30, 50, 60) aus der Hülse (40) derart entfernbar ist, dass, wenn der Dilatator (30, 50, 60) aus der Hülse entfernt wird, die Hülse (40) eine einheitliche und gleichbleibende Steifheit über seine gesamte Länge behält; und dass
die Steifheitsabschnitte weniger steif werden, wenn sie sich dem distalen Ende (33) nähern.

2. Anordnung nach Anspruch 1, wobei der Schaft (32, 51, 61) des Dilatators (30, 50, 60) sich an einem Punkt entlang des Schafts (32, 51, 61) zu dem distalen Ende (33) verjüngt.

3. Anordnung nach Anspruch 1 oder 2, wobei das distale Ende des Schafts (32, 57, 61) des Dilatators (30, 50, 60) mindestens einen röntgendichten Marker enthält.

4. Anordnung nach einem vorhergehenden Anspruch, wobei das distale Ende des Schafts (32, 51, 61) des Dilatators (30, 50, 60) so geformt ist, dass es eine einfachere Gefäßauswahl ermöglicht.

5. Anordnung nach einem der Ansprüche 1 bis 4, wobei der Schaft (32, 51, 61) des Dilatators (30, 50, 60) einen ersten Steifheitsabschnitt (51a, 61a), einen zweiten Steifheitsabschnitt (51b, 61b) und einen dritten Steifheitsabschnitt (51c, 61c) aufweist.

6. Anordnung nach Anspruch 5, wobei der dritte Steifheitsabschnitt (51c, 61c) 15 cm von einem distalen Ende der Hülse (40) beginnt und sich über das distale Ende der Hülse (40) hinaus erstreckt.

7. Anordnung nach Anspruch 5 oder 6, wobei (1) der erste Steifheitsabschnitt (51a, 61a) aus einem Gemisch von 0-95 % eines schlagfesten Polypropylen-Copolymers und 5-100 % eines Hochleistungselastomers hergestellt ist, (2) der zweite Steifheitsabschnitt (51b, 61b) aus einem Gemisch von 0-95 % eines schlagfesten Polypropylen-Copolymers und 5-100 % eines Hochleistungselastomers hergestellt ist und (3) der dritte Steifheitsabschnitt (51c, 61c) aus einem Gemisch von 0-95 % eines schlagfesten Polypropylen-Copolymers und 5-100 % eines Hochleistungselastomers hergestellt ist.

8. Anordnung nach einem der Ansprüche 5 bis 7, wobei (1) der erste Steifheitsabschnitt (51a, 61a) 25-90 cm ist, (2) der zweite Steifheitsabschnitt (51b, 61b) 1-70 cm ist und (3) der dritte Steifheitsabschnitt (51c, 61c) 1-70 cm ist.

9. Anordnung nach einem vorhergehenden Anspruch, wobei die Steifheitsabschnitte (32a, 32b, 51a-c, 61a-c) miteinander verbunden sind, wodurch eine variierende Steifheit und/oder Flexibilität in dem Dilatator (30, 50, 60) erzeugt wird.

10. Anordnung nach Anspruch 9, wobei die Steifheitsabschnitte (32a, 32b, 51a-51c, 61a-61c) mit HF-Energie verschmolzen werden.

11. Anordnung nach einem vorhergehenden Anspruch, wobei der Schaft (32, 51, 61) des Dilatators (30, 50, 60) einen weichsten Abschnitt an dem distalen Ende (33) aufweist und zu einem steifsten Abschnitt an dem proximalen Ende (34) übergeht.

12. Anordnung nach einem vorhergehenden Anspruch, wobei die Steifheitsabschnitte (32a, 32b, 51a-c, 61a-c) unterschiedliche Materialgemische und/oder -qualitäten aufweisen.

13. Anordnung nach einem vorhergehenden Anspruch, die weiterhin Folgendes umfasst:
eine Spitze (62), die mit dem distalen Ende (33) des Schafts (32, 51, 61) des Dilatators (30, 50, 60) verbunden ist.

14. Anordnung nach einem vorhergehenden Anspruch, die weiterhin Folgendes umfasst:
mindestens eine Öffnung (71-78, 81-86), die sich an dem distalen Ende (33) des Schafts (32, 51, 61) des Dilatators (30, 50, 60) befindet, wobei die mindestens eine Öffnung das Abgeben von Kontrastmitteln an das distale Ende unterstützt.

15. Anordnung nach einem vorhergehenden Anspruch, die einen Führungsdraht (20) umfasst.

16. Anordnung nach einem vorhergehenden Anspruch, wobei das distale Ende (33) des Dilatators (30, 50, 60) sich über das distale Ende der Hülse (40) hinaus erstreckt.

17. Anordnung nach einem vorhergehenden Anspruch, wobei das Lumen des Dilatators (30, 50, 60) durch den Dilatator (30, 50, 60) von dem proximalen Ende zu dem distalen Ende (33) verläuft.

18. Anordnung nach einem der Ansprüche 15 bis 17, wobei der Führungsdraht (20) sich durch das Lumen des Dilatators (30, 50, 60) erstreckt.

19. Anordnung nach einem der Ansprüche 15 bis 18, wobei der Führungsdraht (20) ein Draht vom Edelstahl- oder Nitinoltyp mit einem Durchmesser von 0,0965 cm (0,038 Zoll) ist.

20. Anordnung nach einem der vorhergehenden Ansprüche, wobei die Hülse (40) 5 cm - 120 cm lang ist.

## Revendications

1. Un ensemble comprenant :
un dilatateur (30, 50, 60) comprenant une tige (32, 51, 61) ayant une extrémité distale (33) et une extrémité proximale (34), la tige (32, 51, 61) ayant au moins deux sections de rigidité (32a, 32b, 51a-c, 61a-c), une lumière traversant la tige (32, 51, 61) de l'extrémité proximale (34) à l'extrémité distale (33) et un moyeu (31) situé sur l'extrémité proximale (34) de la tige (32, 51, 61) ;
et
une gaine (40) ayant une extrémité distale, une extrémité proximale et une lumière ;
dans lequel le dilatateur (30, 50, 60) s'étend à travers la lumière de la gaine (40) ;
**caractérisé en ce que** le dilatateur (30, 50, 60) peut être retiré de la gaine (40) de telle sorte que, lorsque le dilatateur (30, 50, 60) est retiré de la gaine, la gaine (40) conserve une rigidité uniforme et constante sur toute sa longueur ; et **en ce que**
les sections de rigidité deviennent moins rigides à mesure qu'elles s'approchent de l'extrémité distale (33).

2. L'ensemble selon la revendication 1 dans lequel la tige (32, 51, 61) du dilatateur (30, 50, 60) s'effile à un certain point le long de la tige (32, 51, 61) vers l'extrémité distale (33).

3. L'ensemble selon la revendication 1 ou la revendication 2 dans lequel l'extrémité distale de la tige (32, 51, 61) du dilatateur (30, 50, 60) contient au moins un marqueur radio-opaque.

4. L'ensemble selon l'une quelconque des revendications précédentes dans lequel l'extrémité distale de la tige (32, 51, 61) du dilatateur (30, 50, 60) est d'une forme permettant une sélection plus facile du récipient.

5. L'ensemble selon l'une quelconque des revendications 1 à 4 dans lequel la tige (32, 51, 61) du dilatateur (30, 50, 60) a une première section de rigidité (51a, 61a), une deuxième section de rigidité (51b, 61b) et une troisième section de rigidité (51c, 61c).

6. L'ensemble selon la revendication 5 dans lequel la troisième section de rigidité (51c, 61c) commence à 15 cm d'une extrémité distale de la gaine (40) et s'étend au-delà de l'extrémité distale de la gaine (40).

7. L'ensemble selon la revendication 5 ou la revendication 6 dans lequel (1) la première section de rigidité (51a, 61a) est réalisée dans un mélange contenant entre 0 et 95% d'un copolymère de polypropylène résistant aux chocs et entre 5 et 100% d'élastomère à haute performance, (2) la deuxième section de rigidité (51b, 61b) est réalisée dans un mélange contenant entre 0 et 95% d'un copolymère de polypropylène résistant aux chocs et entre 5 et 100% d'élastomère à haute performance et (3) la troisième section de rigidité (51c, 61c) est réalisée avec entre 0 et 95% d'un copolymère de polypropylène résistant aux chocs et avec entre 5 et 100% d'élastomère à haute performance.

8. L'ensemble selon l'une quelconque des revendications 5 à 7 dans lequel (1) la première section de rigidité (5 1 a, 61a) fait entre 25 et 90 cm, (2) la deuxième section de rigidité (51b, 61b) fait entre 1 et 70 cm et (3) la troisième section de rigidité (51c, 61 c) fait entre 1 et 70 cm.

9. L'ensemble selon l'une quelconque des revendications précédentes dans lequel les sections de rigidité (32a, 32b, 51a-c, 61 a-c) sont jointes ensemble, créant ainsi une rigidité et/ou une flexibilité qui varient dans le dilatateur (30, 50, 60).

10. L'ensemble selon la revendication 9 dans lequel les sections de rigidité (32a, 32b, 51a-c, 61a-c) sont fusionnées avec de l'énergie RF.

11. L'ensemble selon l'une quelconque des revendications précédentes dans lequel la tige (32, 51, 61) du dilatateur (30, 50, 60) a une section plus souple à l'extrémité distale (33) et des transitions vers une section plus rigide à l'extrémité proximale (34).

12. L'ensemble selon l'une quelconque des revendications précédentes dans lequel les sections de rigidité (32a, 32b, 51 a-c, 61 a-c) ont des mélanges et/ou qualités de matériaux différents.

13. L'ensemble selon l'une quelconque des revendications précédentes comprenant en outre :
une pointe (62) raccordée à l'extrémité distale (33) de la tige (32, 51, 61) du dilatateur (30, 50, 60).

14. L'ensemble selon l'une quelconque des revendications précédentes comprenant en outre :
au moins une ouverture (71-78, 81-86) située sur l'extrémité distale (33) de la tige (32, 51, 61) du dilatateur (30, 50, 60), la ou les ouvertures aidant la distribution de l'agent de contraste à l'extrémité distale.

15. L'ensemble selon l'une quelconque des revendications précédentes comprenant un fil de guidage (20).

16. L'ensemble selon l'une quelconque des revendications précédentes dans lequel l'extrémité distale (33) du dilatateur (30, 50, 60) s'étend au-delà de l'extrémité distale de la gaine (40).

17. L'ensemble selon l'une quelconque des revendications précédentes dans lequel la lumière du dilatateur (30, 50, 60) traverse le dilatateur (30, 50, 60) de l'extrémité proximale à l'extrémité distale (33).

18. L'ensemble selon l'une quelconque des revendications 15 à 17 dans lequel le fil de guidage (20) s'étend à travers la lumière du dilatateur (30, 50, 60).

19. L'ensemble selon l'une quelconque des revendications 15 à 18 dans lequel le fil de guidage (20) est un fil en acier inoxydable ou en nitinol d'un diamètre de 0,0965 cm (0,038").

20. L'ensemble selon l'une quelconque des revendications précédentes dans lequel la gaine (40) fait entre 5 cm et 120 cm de long.
